# EUROPEAN PATENT APPLICATION

(11) **EP 4 556 074 A1**
(43) Date of publication of application: **21.05.2025**
(21) Application number: 23839453.0
(22) Date of filing: 27.06.2023
(51) Int. Cl.: A61Q 1/00, A61Q 19/00, A61Q 5/00, A61K 8/06, A61K 8/31, A61K 8/44, A61K 8/92

(54) **OIL-IN-WATER EMULSION COMPOSITION**

(30) Priority: 11.07.2022 JP 2022111422
(71) Applicant: Shiseido Company, Ltd., Chuo-ku Tokyo 104-0061 (JP)
(72) Inventor: KOMAI, Ryota, Tokyo 104-0061 (JP)
(74) Representative: Ter Meer Steinmeister & Partner
(86) International application number: PCT/JP2023/023703
(87) International publication number: WO 2024/014279

(57) **Abstract**

One aspect of the invention is an oil-in-water emulsion composition in which oil-based particles that contain solid oil, liquid oil, and powder are dispersed in an aqueous phase. In the oil-in-water emulsion composition, the solid oil consists of naturally derived wax having a melting point of 50°C or more, the liquid oil contains polar oil and hydrocarbon oil having a branched structure, and a mass ratio of the naturally derived wax to the hydrocarbon oil is 0.01 to 10.

## Description

### TECHNICAL FIELD

The present invention relates to an oil-in-water emulsion composition.

### BACKGROUND OF THE INVENTION

Oil-in-water emulsion compositions in which oil-based particles are dispersed in an aqueous phase prevent moisture from evaporating by covering the skin surface with an oil film, protect the skin from dryness, and impart moisturizing effects to the skin. Therefore, oil-in-water emulsion compositions are applied to various cosmetics. Oil-in-water emulsion compositions contain a variety of ingredients in order to obtain desired quality characteristics.

For example, Patent Document 1 discloses an oil-in-water makeup cosmetic containing fatty acid soap, silicone oil, batyl alcohol, and water-swelling clay minerals. It is also described that the oil-in-water makeup cosmetic contains an aqueous phase and an oil phase, wherein the oil phase may include, for example, hydrocarbons, waxes, and the like, regardless of the composition of the oil phase such as animal oils, vegetable oils, and synthetic oils, and the properties of the oil phase such as solid, semi-solid, and liquid oils.

### RELATED-ART DOCUMENT

### Patent Document

Patent Document 1: Japanese Unexamined Patent Application Publication No. 2018-16587

### SUMMARY OF THE INVENTION

### Problems to be Solved by the Invention

In recent years, oil-in-water emulsion compositions containing naturally derived ingredients have been required due to the growing trend toward natural products. However, naturally derived solid oil contains a large amount of impurities and is more difficult to dissolve in other components of the oil phase than a number of synthetic solid oils, and precipitates may occur.

One aspect of the present invention is to provide an oil-in-water emulsion composition containing naturally derived solid oil and capable of successfully dissolving the naturally derived solid oil in an oil phase.

### Means for Solving the Problems

One aspect of the present invention is an oil-in-water emulsion composition in which oil-based particles that contain solid oil, liquid oil, and powder are dispersed in an aqueous phase. In the oil-in-water emulsion composition, the solid oil consists of naturally derived wax having a melting point of 50°C or more, the liquid oil contains polar oil and hydrocarbon oil having a branched structure, and a mass ratio of the naturally derived wax to the hydrocarbon oil is 0.01 to 10.

### Effects of the Invention

According to one aspect of the present invention, an oil-in-water emulsion composition contains naturally derived solid oil, and the naturally derived solid oil can be successfully dissolved in an oil phase.

### DETAILED DESCRIPTION OF THE INVENTION

Embodiments of the present invention will be described in detail below. Embodiments of the present invention are not limited by the following description, and may be modified as needed without departing from the scope of the present invention. In the present specification, "X to Y" indicating a numerical range is meant to include a lower limit and an upper limit that are given as numerical values before and after "to", unless otherwise noted.

In the oil-in-water emulsion composition according to the present embodiment, oil-based particles containing solid oil, liquid oil, and powder are dispersed in an aqueous phase. That is, the oil phase contains the solid oil, the liquid oil, and the powder. The solid oil consists of naturally derived wax having a melting point of 50°C or more, and the liquid oil contains polar oil and hydrocarbon oil having a branched structure, and a mass ratio of the wax to the hydrocarbon oil is 0.01 to 10. In the present specification, the term "solid oil" means solid or semi-solid oil at room temperature (25°C), and the term "liquid oil" means liquid oil at room temperature (25°C) .

With this configuration, the oil-in-water emulsion composition can contain the naturally derived solid oil, decrease precipitates derived from the naturally derived solid oil, and successfully dissolve the naturally derived solid oil in the oil phase.

The average particle size of the oil-based particles may be 50 µm to 10 mm. When the average particle size of the oil-based particles is 50 µm to 10 mm, the oil-in-water emulsion composition can give a fresh and refreshing feel to a user when applied to the skin and becomes moist after a while. In addition, the oil-in-water emulsion composition can give a sense of beauty to a user because the oil-based particles of a visible size are dispersed in the aqueous phase.

The components included in the oil-in-water emulsion composition of the present embodiment will be described below in detail.

The solid oil consists of the naturally derived wax having a melting point of 50°C or more. Examples of the naturally derived wax having a melting point of 50°C or more include carnauba wax (melting point: 82 to 86°C), candelilla wax (melting point: 68 to 72°C), beeswax (melting point: 62 to 65°C), rice bran oil (melting point: 70 to 83°C), and the like. Among these solid oils, one type may be used alone or two or more types may be used in combination.

When the solid oil consists of the naturally derived wax having a melting point of 50°C or more, a film consisting of the naturally derived wax is formed on the outside of the oil-based particles, and adhesion between the oil-based particles can be minimized. Therefore, the oil-based particles can be stably dispersed in the aqueous phase. The melting point of the naturally derived wax is preferably 60°C or more.

The naturally derived wax having a melting point of 50°C or more is preferably candelilla wax or carnauba wax. When the wax is candelilla wax or carnauba wax, the naturally derived solid oil can be more successfully dissolved in the oil phase. In addition, because the oil-based particles can be made to have an appropriate hardness, the oil-in-water emulsion composition can give a user a feeling that the skin is thickly protected when applied to the skin.

The content of the naturally derived wax having a melting point of 50°C or more is preferably 0.01 mass% to 5 mass%, more preferably 0.1 mass% to 3 mass%, and further more preferably 0.3 mass% to 2 mass% with respect to the oil-in-water emulsion composition. When the content of the naturally derived wax having a melting point of 50°C or more is 0.01 mass% to 5 mass% with respect to the oil-in-water emulsion composition, the naturally derived solid oil can be more successfully dissolved in the oil phase.

The mass ratio of the naturally derived wax having a melting point of 50°C or more to the hydrocarbon oil is more preferably 0.05 to 2, and further more preferably 0.1 to 1. When the mass ratio of the naturally derived wax having a melting point of 50°C or more to the hydrocarbon oil is 0.01 to 10, the naturally derived solid oil can be more successfully dissolved in the oil phase.

The liquid oil contains polar oil and hydrocarbon oil having a branched structure. The polar oil may be any polar oil normally blended in cosmetics, and the like, and is not particularly limited. Examples of the polar oil include diisopropyl sebacate, pentaerythrityl tetraethylhexanoate, cetyl ethylhexanoate, jojoba oil, phytosteryl/octyldodecyl lauroyl glutamate, triisostearin, glyceryl diisostearate, triethylhexanoin, phytosteryl/behenyl dimer dilinoleate, phytosteryl/isostearyl/cetyl/stearyl/isostearyl/cetyl/s tearyl/behenyl dimer dilinoleate, isopropyl palmitate, phytosteryl macadamiate, pentaerythrityl tetrabehenate/benzoate/ethylhexanoate, ethylhexyl palmitate, myristyl myristate, isopropyl myristate, tripropylene glycol dipivalate, isodecyl neopentanoate, and the like.

The content of the polar oil is preferably equal to or less than the content of the hydrocarbon oil in the oil-in-water emulsion composition. When the content of the polar oil is equal to or less than the content of the hydrocarbon oil in the oil-in-water emulsion composition, the naturally derived solid oil can be successfully dissolved in the oil phase. At the same time, because the oil-based particles can be made to have an appropriate hardness, the oil-in-water emulsion composition can exhibit good usability. In addition, the formability and stability of the oil-based particles can be improved.

Examples of the hydrocarbon oil having a branched structure include squalane, hydrogenated polyisobutene, hydrogenated polydecene, isohexadecane, and the like. The squalane includes naturally derived squalane such as animal squalane, vegetable squalane, and the like, and synthetic squalane.

The carbon number of the hydrocarbon oil having a branched structure is preferably 10 to 40. When the carbon number of the hydrocarbon oil having a branched structure is 10 to 40, the naturally derived solid oil can be more successfully dissolved in the oil phase. Further, the strength of the oil-based particles can be improved, and the oil-based particles can be stably dispersed in the aqueous phase. In addition, the oil-in-water emulsion composition can give a feeling of good skin compatibility to a user when applied to the skin.

The hydrocarbon oil having a branched structure is preferably naturally derived squalane. Accordingly, the oil-in-water emulsion composition contains the naturally derived solid oil and the naturally derived liquid oil, and the naturally derived solid oil can be successfully dissolved in the oil phase.

The content of the hydrocarbon oil having a branched structure is preferably 0.01 mass% to 10 mass%, more preferably 0.5 mass% to 5 mass%, and further more preferably 0.1 mass% to 3 mass% with respect to the oil-in-water emulsion composition. When the content of the hydrocarbon oil having a branched structure is 0.01 mass% to 10 mass% with respect to the oil-in-water emulsion composition, the naturally derived solid oil can be successfully dissolved in the oil phase.

The liquid oil may contain a small amount of silicone oil in addition to the polar oil and the hydrocarbon oil. Examples of the silicone oil include chain silicones such as dimethyl polysiloxane (dimethicone), methylphenyl polysiloxane (phenylmethicone), methyl hydrogen polysiloxane, and the like; cyclic silicones such as decamethylcyclopentasiloxane, dodecamethylcyclohexasiloxane, and the like; amino-modified silicone oil; polyether-modified silicone oil; carboxy-modified silicone oil; alkyl-modified silicone oil; ammonium-salt-modified silicone oil; fluorine-modified silicone oil; and the like.

When the liquid oil contains a small amount of silicone oil, the content of the silicone oil is preferably 5 mass% or less, more preferably 3 mass% or less, further more preferably 2 mass% or less, further more preferably 1 mass% or less, and further more preferably 0.1 mass% or less with respect to the oil-in-water emulsion composition. It is particularly preferable that the liquid oil does not contain silicone oil.

The content of the silicone oil is preferably 30 mass% or less, more preferably 20 mass% or less, and further more preferably 10 mass% or less with respect to the total amount of the solid oil and the liquid oil. It is particularly preferable that the liquid oil does not contain silicone oil.

Typical oil-in-water emulsion compositions contain silicone oil in the liquid oil to improve stability or feeling of use. By the oil-in-water emulsion composition according to the present embodiment not containing silicone oil in the liquid oil, it is possible to provide a silicone-free oil-in-water emulsion composition, which has been in increasing demand in recent years.

Because the powder is contained in the oil-based particles, it may be hydrophobic powder. The hydrophobic powder includes hydrophobic powder whose surface has not been subjected to hydrophilic treatment, and hydrophobic or hydrophilic powder whose surface has been treated with a hydrophobic substance.

The powder is not particularly limited as long as it can be incorporated into external skin preparations such as cosmetics. Examples of the powder include inorganic powders such as talc, mica, kaolin, mica, silk mica (sericite), muscovite, phlogopite, synthetic mica, red mica, biotite, lithia mica, vermiculite, magnesium carbonate, calcium carbonate, aluminum silicate, barium silicate, calcium silicate, magnesium silicate, strontium silicate, tungstate metal salt, magnesium, spherical silica, zeolite, barium sulfate, calcined calcium sulfate (calcined gypsum), calcium phosphate, fluorapatite, hydroxyapatite, ceramic powder, metal soaps (zinc myristate, calcium palmitate, calcium stearate, aluminum stearate, and the like), and boron nitride; spherical organic powders such as polyamide spherical resin powder (nylon spherical powder), spherical polyethylene, crosslinked methyl poly(meth)acrylate spherical resin powder, spherical polyester, crosslinked polystyrene spherical resin powder, styrene and acrylic acid copolymer spherical resin powder, benzoguanamine spherical resin powder, polytetrafluoroethylene spherical powder, and spherical cellulose; inorganic white pigments such as titanium dioxide, and zinc oxide; inorganic red pigments such as iron oxide (Bengala), and iron titanate; inorganic brown pigments such as γ-iron oxide; inorganic yellow pigments such as yellow iron oxide and ochre; inorganic black pigments such as black iron oxide, carbon black, and lower titanium oxide; inorganic purple pigments such as mango violet and cobalt violet; inorganic green pigments such as chromium oxide, chromium hydroxide, and cobalt titanate; inorganic blue pigments such as ultramarine blue and iron blue; pearl pigments such as titanium oxide-coated mica, titanium oxide-coated bismuth oxychloride, titanium oxide-coated talc, colored titanium oxide-coated mica, bismuth oxychloride, borosilicate (Ca/Al), fish scale foil; metallic powder pigments such as aluminum powder and copper powder; color materials in red, yellow, orange, yellow, green, blue, or the like, or color materials (organic pigments) obtained by laking these materials with zirconium, barium, aluminum, or the like; natural pigments such as chlorophyll, β-carotene, and the like.

Examples of the powder whose surface has been treated with a hydrophobic substance include ultraviolet scattering agents such as aluminum stearate-coated titanium oxide, magnesium isostearate-coated titanium oxide, dextrin palmitate-coated zinc oxide; hydrophobized talc; and the like.

The powder preferably contains an amino acid, an amino acid derivative, or powder coated with the amino acid or the amino acid derivative. Examples of the amino acid include glutamic acid and the like. Examples of the amino acid derivative include amino acid-based surfactants such as lauroyl lysine, potassium cocoyl glutamate, sodium cocoylglycine, and the like. Examples of the powder coated with the amino acid or the amino acid derivative include sodium dilauramidoglutamide lysine-coated iron oxide (red), sodium dilauramidoglutamide lysine-coated titanium oxide, and powder whose surface has been treated with amino acid derivatives such as disodium stearoyl glutamate, magnesium palmitoyl glutamate, disodium cocoyl glutamate, and the like. When the powder contains the amino acid, the amino acid derivative, or the powder coated with the amino acid or the amino acid derivative, the oil-in-water emulsion composition can more successfully dissolve the naturally derived solid oil in the oil phase.

The amino acid derivative is preferably lauroyl lysine. When the amino acid derivative is lauroyl lysine, the oil-based particles of the oil-in-water emulsion composition contain the naturally derived solid oil and the naturally derived powder, and the naturally derived solid oil can be more successfully dissolved in the oil phase. In addition, because lauroyl lysine has a relatively small coefficient of friction with respect to the other powders mentioned above, the oil-in-water emulsion composition can give a smooth and silky feeling to a user when applied to the skin.

The average primary particle size of the powder is preferably 0.01 µm to 15 um, more preferably 0.05 µm to 12 µm, and further more preferably 0.1 µm to 10 µm. When the average primary particle size of the powder is 0.01 µm to 15 µm, the naturally derived solid oil can be more successfully dissolved in the oil phase.

The content of the powder is preferably 0.01 mass% to 30 mass%, more preferably 0.1 mass% to 20 mass%, and further more preferably 0.5 mass% to 10 mass% with respect to the total amount of the naturally derived wax having a melting point of 50°C or more and the hydrocarbon oil having a branched structure. When the content of the powder is 0.01 mass% to 30 mass% with respect to the total amount of the naturally derived wax having a melting point of 50°C or more and the hydrocarbon oil having a branched structure, the naturally derived solid oil can be more successfully dissolved in the oil phase.

When the powder is an ultraviolet scattering agent, the upper limit of the content of the ultraviolet scattering agent is preferably 1 mass% with respect to the oil-in-water emulsion composition. Specifically, the content of the ultraviolet scattering agent is preferably 0.0001 mass% to 1 mass% with respect to the oil-in-water emulsion composition. When the upper limit of the content of the ultraviolet scattering agent is 1 mass% with respect to the oil-in-water emulsion composition, the naturally derived solid oil can be more successfully dissolved in the oil phase, and the oil-in-water emulsion composition can exhibit an ultraviolet protection effect.

The aqueous phase contains a typical aqueous medium and serves as a dispersion medium for the oil-based particles. Examples of the aqueous medium include water, ethanol, and the like. Among these aqueous media, one type may be used alone or two or more types may be used in combination.

The content of the aqueous medium is preferably 50 mass% to 99 mass%, more preferably 60 mass% to 98 mass%, and further more preferably 70 mass% to 95 mass% with respect to the oil-in-water emulsion composition.

The oil-in-water emulsion composition may contain, in addition to the above essential components, other components that can be incorporated into external skin preparations such as cosmetics, within a range that does not interfere with the effect of the present invention.

Examples of the other components that can be incorporated into the oil-in-water emulsion composition include, but are not limited to, water-soluble thickeners; oil-soluble thickeners; water-soluble agents such as arbutin, ascorbic acid glucoside, tranexamic acid, and 4-methoxysalicylate; moisturizers such as glycerin, dipropylene glycol, 1,3-butylene glycol, and propanediol; water-soluble ultraviolet absorbers such as dimorpholinopyridazinone; chelating agents such as sodium edetate and potassium hydroxide; pH adjusters such as citric acid and sodium citrate; preservatives such as paraben and phenoxyethanol; pigments, fragrances, surfactants, and the like.

Examples of the water-soluble thickener include, but are not particularly limited to, plant-based polymers such as gum arabic, tragacanth gum, galactan, carob gum, guar gum, gum karaya, carrageenan, pectin, agar, pyrus cydonia seed (pyrus cydonia), and algae colloid (extract of Phaeophyta); microbial-based polymers such as dextran, succinoglycan, and pullulan; animal-based polymers such as collagen, casein, albumin, and gelatin; cellulose-based polymers such as methyl cellulose, nitrocellulose, ethyl cellulose, methyl hydroxypropyl cellulose, hydroxy ethyl cellulose, sodium cellulose sulfate, hydroxypropyl cellulose, sodium carboxymethyl cellulose, crystalline cellulose, and cellulose powder; alginic acid-based polymers such as sodium alginate, and propylene glycol alginate; vinyl-based polymers such as polyvinyl alcohol, polyvinyl methyl ether, polyvinylpyrrolidone, carboxy vinyl polymers, and alkyl-modified carboxy vinyl polymers; acrylic-based polymers such as polyoxyethylene-based polymers, polyoxyethylene/polyoxypropylene copolymer-based polymers, sodium polyacrylate, polyethyl acrylate, polyacrylamide, and (ammonium acryloyl dimethyltaurate/VP) copolymer; and inorganic-based water-soluble polymers such as polyethyleneimine, cationic polymers, bentonite, aluminum magnesium silicate, laponite, hectorite, and silicic anhydride; and the like.

As the water-soluble thickener, the alkyl-modified carboxy vinyl polymers are particularly suitable because they exert an action to minimize aggregation and coalescence of the oil-based particles based on their interfacial activity. The alkyl-modified carboxy vinyl polymers are also called acrylic acid-alkyl methacrylate copolymers ((acrylates/alkyl acrylate (C10-30)) crosspolymers) and are commercially available under the trade names of, for example, CARBOPOL (registered trademark) 1342, PEMULEN (registered trademark) TR-1, and PEMULEN (registered trademark) TR-2 (manufactured by BF Goodrich).

The oil-soluble thickener can be adjusted so as to reduce the solidifying power of the solid oil that constitutes the oil-based particles and to obtain an appropriate hardness. Examples of the oil-soluble thickener include, but are not limited to, dextrin fatty acid esters, lime soaps, lipophilic bentonites, sucrose fatty acid esters, benzylidene derivatives of sorbitol, and the like.

Examples of the dextrin fatty acid esters include dextrin palmitate, dextrin oleate, dextrin stearate, and the like.

Examples of the lime soaps include aluminum stearate, magnesium stearate, zinc myristate, and the like, in which hydroxyl groups remain.

Examples of the lipophilic bentonites include dimethylbenzyldodecylammonium montmorillonite, dimethyldioctadecylammonium montmorillonite, and the like.

Examples of the sucrose fatty acid esters include those in which three or less of eight hydroxyl groups are esterified with higher fatty acids, and the higher fatty acids are stearic acid and palmitic acid.

Examples of the benzylidene derivatives of sorbitol include monobenzylidene sorbitol, dibenzylidene sorbitol, and the like.

Among these, as the oil-soluble thickener, dextrin palmitate is particularly preferred from the viewpoint of stability and usability. The dextrin fatty acid esters are not particularly limited, but for example, those commercially available under trade names of Rheopearl (registered trademark) KL and Rheopearl (registered trademark) KE (manufactured by Chiba Flour Milling Co., Ltd.) can be used.

The oil-in-water emulsion composition may be prepared by, for example, the following methods. First, aqueous phase components are mixed, heated to the melting point or higher of the solid oil to be blended, and oil-phase components (contains solid oil, liquid oil, and powder) heated and mixed at the same temperature as the aqueous phase components are added to the aqueous phase components under stirring. At this time, in the oil-in-water emulsion composition according to the present embodiment, the naturally derived solid oil is successfully dissolved in the oil phase, and a uniform oil phase is obtained. The stirring can be performed using a propeller or paddle mixer at a speed of 10 to 1,500 rpm, preferably about 20 to 300 rpm. When stirring, it is not preferable to use a homomixer because the mixture becomes fine. Then, by cooling while stirring, the oil-in-water emulsion composition can be prepared.

The oil-in-water emulsion composition produced in this manner has oil-based particles having an average particle size of 50 µm to 10 mm dispersed in the aqueous phase. The oil-based particles according to the present embodiment are considered to have a capsule structure in which the inner layer is substantially composed of powder and liquid oil and the surrounding area is covered with solid oil (the outer layer). In addition, the solid oil that forms the outer layer crystallizes, resulting in an appropriate hardness and a unique feeling of use.

In the composition according to the present invention, the oil-based particles of the above structure can be formed without using a surfactant, but the composition has a structure in which the powder exists inside the oil-based particles (the inner layer) and the solid oil exists at the interface between the aqueous phase and the oil phase, and is clearly distinguished from a Pickering emulsion in which the powder exists at the oil-water interface.

The oil-in-water emulsion composition according to the present invention is suitable for use as a base for cosmetics. Examples of cosmetics using the base include water-based cosmetics, gel cosmetics, emulsified cosmetics, and the like. Specific applications include facial, body, or hair cosmetics such as lotions, gels, emulsions, creams, or packs.

### Examples

Embodiments will be described more specifically below with Examples and Comparative Examples, but the embodiments are not limited by these Examples and Comparative Examples.

The oil-in-water emulsion compositions were prepared by the following methods with the compositions presented in Tables 1 to 5, and the obtained oil-in-water emulsion compositions were evaluated by the following methods.

### <Preparation of Oil-in-Water Emulsion Composition>

With the compositions presented in Tables 1 to 5, the aqueous phase components were mixed, heated to the melting point or higher of the solid oil to be blended, and the oil-phase components (contains solid oil, liquid oil, and powder) heated and mixed at the same temperature as the aqueous phase components were added to the aqueous phase components under stirring. Then, by cooling while stirring, each oil-in-water emulsion composition was obtained.

For each oil-in-water emulsion composition prepared, the solubility of the solid oil at the time of preparation of the oil-in-water emulsion composition and the feeling of use of the oil-in-water emulsion composition (skin compatibility) were evaluated based on the following evaluation criteria.

### <Evaluation of Solubility of Solid Oil>

After mixing and heating the oil phase components (contains solid oil, liquid oil, and powder) at the time of preparation of the oil-in-water emulsion composition, the uniformity of the oil phase was visually checked. C or higher was determined as acceptable, and the evaluation results are presented in Tables 1 to 5.

### [Evaluation Criteria for Average Points]

A: After mixing and heating, the oil phase dissolved quickly and was uniform without precipitates.
B: After mixing and heating, the oil phase dissolved and was uniform without precipitates.
C: After mixing and heating for a long time, the oil phase dissolved and was uniform without precipitates.
D: The oil phase did not dissolve after mixing and heating.

### <Evaluation of Feeling of Use>

Practical use tests were conducted by a professional panel, and the feeling of use was evaluated based on the following criteria from the viewpoint of the skin compatibility. A was determined as acceptable, and the evaluation results are presented in Tables 1 to 5.

### [Evaluation criteria]

A: Excellent
B: Slightly inferior

**[Table 1]**

| | | | EXAMPLE 1 | EXAMPLE 2 | EXAMPLE 3 | EXAMPLE 4 | EXAMPLE 5 | EXAMPLE 6 | EXAMPLE 7 |
|---|---|---|---|---|---|---|---|---|---|
| WATER | WATER | | BALANCE | BALANCE | BALANCE | BALANCE | BALANCE | BALANCE | BALANCE |
| SOLID OIL | (B) | CANDELILLA WAX | 0.60 | 0.60 | 0.60 | 0.60 | - | - | - |
| | | CARNAUBA WAX | - | - | - | - | 0.60 | - | - |
| | | BEESWAX | - | - | - | - | - | 0.60 | - |
| | | RICE BRAN OIL | - | - | - | - | - | - | 0.60 |
| LIQUID OIL | PENTAERYTHRITYL TETRAETHYLHEXANOATE | | 0.75 | 0.75 | 0.75 | 0.75 | 0.75 | 0.75 | 0.75 |
| | (A) | SQUALANE | 1.20 | - | - | - | 1.20 | 1.20 | 1.20 |
| | | HYDROGENATED POLYISOBUTENE | - | 1.20 | - | - | - | - | - |
| | | HYDROGENATED POLYDECENE | - | - | 1.20 | - | - | - | - |
| | | ISOHEXADECANE | - | - | - | 1.20 | - | - | - |
| POWDER | (C) | LAUROYL LYSINE | 0.10 | 0.10 | 0.10 | 0.10 | 0.10 | 0.10 | 0.10 |
| | | HYDROPHOBIZED TALC | - | - | - | - | - | - | - |
| | | CALCIUM STEARATE | - | - | - | - | - | - | - |
| | | ALUMINUM STEARATE-COATED TITANIUM OXIDE | | | | | | | |
| | | MAGNESIUM ISOSTEARATE-COATED TITANIUM OXIDE | - | - | - | - | - | - | - |
| | | SODIUM DILAURAMIDOGLUTAMIDE LYSINE-COATED TITANIUM OXIDE | - | - | - | - | - | - | - |
| | | DEXTRIN PALMITATE-COATED ZINC OXIDE | | | | | | | |
| | GLYCERIN | | 4.00 | 4.00 | 4.00 | 4.00 | 4.00 | 4.00 | 4.00 |
| | 1,3-BUTYLENE GLYCOL | | 2.00 | 2.00 | 2.00 | 2.00 | 2.00 | 2.00 | 2.00 |
| | PROPANEDIOL | | 7.00 | 7.00 | 7.00 | 7.00 | 7.00 | 7.00 | 7.00 |
| | CARBOXY VINYL POLYMER | | 0.12 | 0.12 | 0.12 | 0.12 | 0.12 | 0.12 | 0.12 |
| OTHERS | (ACRYLATES/ALKYL ACRYLATE (C10-30)) CROSSPOLYMER | | 0.06 | 0.06 | 0.06 | 0.06 | 0.06 | 0.06 | 0.06 |
| | (AMMONIUM ACRYLOYL DIMETHYLTAURATE/VP) COPOLYMER | | 0.60 | 0.60 | 0.60 | 0.60 | 0.60 | 0.60 | 0.60 |
| | POTASSIUM HYDROXIDE | | 0.10 | 0.10 | 0.10 | 0.10 | 0.10 | 0.10 | 0.10 |
| | PHENOXYETHANOL | | 0.50 | 0.50 | 0.50 | 0.50 | 0.50 | 0.50 | 0.50 |
| | EDTA-2Na | | 0.01 | 0.01 | 0.01 | 0.01 | 0.01 | 0.01 | 0.01 |
| TOTAL (MASS %) | | | 100 | 100 | 100 | 100 | 100 | 100 | 100 |
| B/A | | | 0.50 | 0.50 | 0.50 | 0.50 | 0.50 | 0.50 | 0.50 |
| C/(A+B) × 100 (%) | | | 5.56 | 5.56 | 5.56 | 5.56 | 5.56 | 5.56 | 5.56 |
| EVALUATION | SOLUBILITY OF SOLID OIL | | A | A | A | A | A | A | A |
| | SKIN COMPATIBILITY | | A | A | A | A | A | A | A |

**[Table 2]**

| | | | EXAMPLE 8 | EXAMPLE 9 | EXAMPLE 10 | EXAMPLE 11 | EXAMPLE 12 | EXAMPLE 13 | COMPARATIVE EXAMPLE 1 |
|---|---|---|---|---|---|---|---|---|---|
| WATER | WATER | | BALANCE | BALANCE | BALANCE | BALANCE | BALANCE | BALANCE | BALANCE |
| SOLID OIL | (B) | CANDELILLA WAX | 0.60 | 0.60 | 0.60 | 0.60 | 0.60 | 0.60 | 0.60 |
| | | CARNAUBA WAX | - | - | - | - | - | - | - |
| | | BEESWAX | - | - | - | - | - | - | - |
| | | RICE BRAN OIL | - | - | - | - | - | - | - |
| LIQUID OIL | PENTAERYTHRITYL TETRAETHYLHEXANOATE | | 0.75 | 0.75 | 0.75 | 0.75 | 0.75 | 0.75 | 0.75 |
| | (A) | SQUALANE | 1.20 | 1.20 | 1.20 | 1.20 | 1.20 | 1.20 | 1.20 |
| | | HYDROGENATED POLYISOBUTENE | - | - | - | - | - | - | - |
| | | HYDROGENATED POLYDECENE | - | - | - | - | - | - | - |
| | | ISOHEXADECANE | - | - | - | - | - | - | - |
| POWDER | (C) | LAUROYL LYSINE | - | - | - | - | - | - | - |
| | | HYDROPHOBIZED TALC | 0.10 | - | - | - | - | - | - |
| | | CALCIUM STEARATE | - | 0.10 | - | - | - | - | - |
| | | ALUMINUM STEARATE-COATED TITANIUM OXIDE | - | - | 0.10 | - | - | - | - |
| | | MAGNESIUM ISOSTEARATE-COATED TITANIUM OXIDE | - | - | - | 0.10 | - | - | - |
| | | SODIUM DILAURAMIDOGLUTAMIDE LYSINE-COATED TITANIUM OXIDE | - | - | - | - | 0.10 | - | - |
| | | DEXTRIN PALMITATE-COATED ZINC OXIDE | - | - | - | - | - | 0.10 | - |
| | GLYCERIN | | 4.00 | 4.00 | 4.00 | 4.00 | 4.00 | 4.00 | 4.00 |
| | 1,3-BUTYLENE GLYCOL | | 2.00 | 2.00 | 2.00 | 2.00 | 2.00 | 2.00 | 2.00 |
| | PROPANEDIOL | | 7.00 | 7.00 | 7.00 | 7.00 | 7.00 | 7.00 | 7.00 |
| | CARBOXY VINYL POLYMER | | 0.12 | 0.12 | 0.12 | 0.12 | 0.12 | 0.12 | 0.12 |
| OTHERS | (ACRYLATES/ALKYL ACRYLATE (C10-30)) CROSSPOLYMER | | 0.06 | 0.06 | 0.06 | 0.06 | 0.06 | 0.06 | 0.06 |
| | (AMMONIUM ACRYLOYL DIMETHYLTAURATE/VP) COPOLYMER | | 0.60 | 0.60 | 0.60 | 0.60 | 0.60 | 0.60 | 0.60 |
| | POTASSIUM HYDROXIDE | | 0.10 | 0.10 | 0.10 | 0.10 | 0.10 | 0.10 | 0.10 |
| | PHENOXYETHANOL | | 0.50 | 0.50 | 0.50 | 0.50 | 0.50 | 0.50 | 0.50 |
| | EDTA-2Na | | 0.01 | 0.01 | 0.01 | 0.01 | 0.01 | 0.01 | 0.01 |
| TOTAL (MASS %) | | | 100 | 100 | 100 | 100 | 100 | 100 | 100 |
| B/A | | | 0.50 | 0.50 | 0.50 | 0.50 | 0.50 | 0.50 | 0.50 |
| C/(A+B) × 100 (%) | | | 5.56 | 5.56 | 5.56 | 5.56 | 5.56 | 5.56 | - |
| EVALUATION | SOLUBILITY OF SOLID OIL | | A | A | A | A | A | A | D |
| | SKIN COMPATIBILITY | | A | A | A | A | A | A | B |

**[Table 3]**

| | | | EXAMPLE 14 | EXAMPLE 15 | EXAMPLE 16 | EXAMPLE 17 | EXAMPLE 18 | EXAMPLE 19 |
|---|---|---|---|---|---|---|---|---|
| WATER | WATER | | BALANCE | BALANCE | BALANCE | BALANCE | BALANCE | BALANCE |
| SOLID OIL | (B) | CANDELILLA WAX | 0.60 | 0.60 | 0.60 | 0.60 | 0.60 | 0.60 |
| | | CARNAUBA WAX | - | - | - | - | - | - |
| | | BEESWAX | - | - | - | - | - | - |
| | | RICE BRAN OIL | - | - | - | - | - | - |
| LIQUID OIL | PENTAERYTHRITYL TETRAETHYLHEXANOATE | | 0.75 | 0.75 | 0.75 | 0.75 | 0.75 | 0.75 |
| | (A) | SQUALANE | 1.20 | 1.20 | 1.20 | 1.20 | 1.20 | 1.20 |
| | | HYDROGENATED POLYISOBUTENE | - | - | - | - | - | - |
| | | HYDROGENATED POLYDECENE | - | - | - | - | - | - |
| | | ISOHEXADECANE | - | - | - | - | - | - |
| POWDER | (C) | LAUROYL LYSINE | 1.00 | 0.50 | 0.05 | 0.01 | 0.001 | 0.0001 |
| | | HYDROPHOBIZED TALC | - | - | - | - | - | - |
| | | CALCIUM STEARATE | - | - | - | - | - | - |
| | | ALUMINUM STEARATE-COATED TITANIUM OXIDE | - | - | - | - | - | - |
| | | MAGNESIUM ISOSTEARATE-COATED TITANIUM OXIDE | - | - | - | - | - | - |
| | | SODIUM DILAURAMIDOGLUTAMIDE LYSINE-COATED TITANIUM OXIDE | - | - | - | - | - | - |
| | | DEXTRIN PALMITATE-COATED ZINC OXIDE | | | | | | |
| | GLYCERIN | | 4.00 | 4.00 | 4.00 | 4.00 | 4.00 | 4.00 |
| | 1,3-BUTYLENE GLYCOL | | 2.00 | 2.00 | 2.00 | 2.00 | 2.00 | 2.00 |
| | PROPANEDIOL | | 7.00 | 7.00 | 7.00 | 7.00 | 7.00 | 7.00 |
| | CARBOXY VINYL POLYMER | | 0.12 | 0.12 | 0.12 | 0.12 | 0.12 | 0.12 |
| OTHERS | (ACRYLATES/ALKYL ACRYLATE (C10-30)) CROSSPOLYMER | | 0.06 | 0.06 | 0.06 | 0.06 | 0.06 | 0.06 |
| | (AMMONIUM ACRYLOYL DIMETHYLTAURATE/VP) COPOLYMER | | 0.60 | 0.60 | 0.60 | 0.60 | 0.60 | 0.60 |
| | POTASSIUM HYDROXIDE | | 0.10 | 0.10 | 0.10 | 0.10 | 0.10 | 0.10 |
| | PHENOXYETHANOL | | 0.50 | 0.50 | 0.50 | 0.50 | 0.50 | 0.50 |
| | EDTA-2Na | | 0.01 | 0.01 | 0.01 | 0.01 | 0.01 | 0.01 |
| TOTAL (MASS %) | | | 100 | 100 | 100 | 100 | 100 | 100 |
| B/A | | | 0.50 | 0.50 | 0.50 | 0.50 | 0.50 | 0.50 |
| C/(A+B) × 100 (%) | | | 55.56 | 27.78 | 2.78 | 0.56 | 0.06 | 0.01 |
| EVALUATION | SOLUBILITY OF SOLID OIL | | C | B | A | A | B | C |
| | SKIN COMPATIBILITY | | A | A | A | A | A | A |

**[Table 4]**

| | | | EXAMPLE 20 | EXAMPLE 21 | EXAMPLE 22 | EXAMPLE 23 | EXAMPLE 24 | EXAMPLE 25 |
|---|---|---|---|---|---|---|---|---|
| WATER | WATER | | BALANCE | BALANCE | BALANCE | BALANCE | BALANCE | BALANCE |
| SOLID OIL | (B) | CANDELILLA WAX | 0.10 | 0.20 | 0.60 | 0.90 | 1.20 | 1.60 |
| | | CARNAUBA WAX | - | - | - | - | - | - |
| | | BEESWAX | - | - | - | - | - | - |
| | | RICE BRAN OIL | - | - | - | - | - | - |
| LIQUID OIL | PENTAERYTHRITYL TETRAETHYLHEXANOATE | | | | | | | |
| | (A) | SQUALANE | 1.70 | 1.60 | 1.20 | 0.90 | 0.60 | 0.20 |
| | | HYDROGENATED POLYISOBUTENE | - | - | - | - | - | - |
| | | HYDROGENATED POLYDECENE | - | - | - | - | - | - |
| | | ISOHEXADECANE | - | - | - | - | - | - |
| POWDER | (C) | LAUROYL LYSINE | 0.10 | 0.10 | 0.10 | 0.10 | 0.10 | 0.10 |
| | | HYDROPHOBIZED TALC | - | - | - | - | - | - |
| | | CALCIUM STEARATE | - | - | - | - | - | - |
| | | ALUMINUM STEARATE-COATED TITANIUM OXIDE | - | - | - | - | - | - |
| | | MAGNESIUM ISOSTEARATE-COATED TITANIUM OXIDE | | | | | | |
| | | SODIUM DILAURAMIDOGLUTAMIDE LYSINE-COATED TITANIUM OXIDE | - | - | - | - | - | - |
| | | DEXTRIN PALMITATE-COATED ZINC OXIDE | | | | | | |
| | GLYCERIN | | 4.00 | 4.00 | 4.00 | 4.00 | 4.00 | 4.00 |
| | 1,3-BUTYLENE GLYCOL | | 2.00 | 2.00 | 2.00 | 2.00 | 2.00 | 2.00 |
| | PROPANEDIOL | | 7.00 | 7.00 | 7.00 | 7.00 | 7.00 | 7.00 |
| | CARBOXY VINYL POLYMER | | 0.12 | 0.12 | 0.12 | 0.12 | 0.12 | 0.12 |
| OTHERS | (ACRYLATES/ALKYL ACRYLATE (C10-30)) CROSSPOLYMER | | 0.06 | 0.06 | 0.06 | 0.06 | 0.06 | 0.06 |
| | (AMMONIUM ACRYLOYL DIMETHYLTAURATE/VP) COPOLYMER | | 0.60 | 0.60 | 0.60 | 0.60 | 0.60 | 0.60 |
| | POTASSIUM HYDROXIDE | | 0.10 | 0.10 | 0.10 | 0.10 | 0.10 | 0.10 |
| | PHENOXYETHANOL | | 0.50 | 0.50 | 0.50 | 0.50 | 0.50 | 0.50 |
| | EDTA-2Na | | 0.01 | 0.01 | 0.01 | 0.01 | 0.01 | 0.01 |
| TOTAL (MASS %) | | | 100 | 100 | 100 | 100 | 100 | 100 |
| B/A | | | 0.06 | 0.13 | 0.50 | 1.00 | 2.00 | 8.00 |
| C/(A+B) × 100 (%) | | | 5.56 | 5.56 | 5.56 | 5.56 | 5.56 | 5.56 |
| EVALUATION | SOLUBILITY OF SOLID OIL | | A | A | A | A | B | B |
| | SKIN COMPATIBILITY | | B | A | A | A | A | B |

**[Table 5]**

| | | | EXAMPLE 26 | EXAMPLE 27 | EXAMPLE 28 | EXAMPLE 29 | EXAMPLE 30 |
|---|---|---|---|---|---|---|---|
| WATER | WATER | | BALANCE | BALANCE | BALANCE | BALANCE | BALANCE |
| SOLID OIL | (B) | CANDELILLA WAX | 0.60 | 0.60 | 0.60 | 0.60 | 0.60 |
| | | CARNAUBA WAX | - | - | - | - | - |
| | | BEESWAX | - | - | - | - | - |
| | | RICE BRAN OIL | - | - | - | - | - |
| LIQUID OIL | PENTAERYTHRITYL TETRAETHYLHEXANOATE | | 0.75 | 0.75 | 0.75 | 0.75 | 0.75 |
| | (A) | SQUALANE | 1.20 | 1.20 | 1.20 | 1.20 | 1.20 |
| | | HYDROGENATED POLYISOBUTENE | - | - | - | - | - |
| | | HYDROGENATED POLYDECENE | - | - | - | - | - |
| | | ISOHEXADECANE | - | - | - | - | - |
| POWDER | (C) | LAUROYL LYSINE | - | - | - | - | - |
| | | HYDROPHOBIZED TALC | - | - | - | - | - |
| | | CALCIUM STEARATE | - | - | - | - | - |
| | | ALUMINUM STEARATE-COATED TITANIUM OXIDE | 1.00 | 0.50 | 0.10 | 0.001 | 0.0001 |
| | | MAGNESIUM ISOSTEARATE-COATED TITANIUM OXIDE | | | | | |
| | | SODIUM DILAURAMIDOGLUTAMIDE LYSINE-COATED TITANIUM OXIDE | | | | | |
| | | DEXTRIN PALMITATE-COATED ZINC OXIDE | | | | | |
| | GLYCERIN | | 4.00 | 4.00 | 4.00 | 4.00 | 4.00 |
| | 1,3-BUTYLENE GLYCOL | | 2.00 | 2.00 | 2.00 | 2.00 | 2.00 |
| | PROPANEDIOL | | 7.00 | 7.00 | 7.00 | 7.00 | 7.00 |
| | CARBOXY VINYL POLYMER | | 0.12 | 0.12 | 0.12 | 0.12 | 0.12 |
| OTHERS | (ACRYLATES/ALKYL ACRYLATE (C10-30)) CROSSPOLYMER | | 0.06 | 0.06 | 0.06 | 0.06 | 0.06 |
| | (AMMONIUM ACRYLOYL DIMETHYLTAURATE/VP) COPOLYMER | | 0.60 | 0.60 | 0.60 | 0.60 | 0.60 |
| | POTASSIUM HYDROXIDE | | 0.10 | 0.10 | 0.10 | 0.10 | 0.10 |
| | PHENOXYETHANOL | | 0.50 | 0.50 | 0.50 | 0.50 | 0.50 |
| | EDTA-2Na | | 0.01 | 0.01 | 0.01 | 0.01 | 0.01 |
| TOTAL (MASS %) | | | 100 | 100 | 100 | 100 | 100 |
| B/A | | | 0.50 | 0.50 | 0.50 | 0.50 | 0.50 |
| C/(A+B) × 100 (%) | | | 55.56 | 27.78 | 5.56 | 0.06 | 0.01 |
| EVALUATION | SOLUBILITY OF SOLID OIL | | C | A | A | B | C |
| | SKIN COMPATIBILITY | | A | A | A | A | A |

As described in Tables 1 to 5, it was confirmed that the oil-in-water emulsion compositions of Examples 1 to 19, 21 to 24, and 26 to 30 had no problems in actual use in the solubility of the solid oil and in the feeling of use. The oil-in-water emulsion compositions of Examples 20 and 25 had no problems in actual use in the solubility of the solid oil, but had a slightly inferior feeling of use. In contrast, the oil-in-water emulsion composition of Comparative Example 1, which did not contain powder in the oil-based particles, did not dissolve even when heated after the oil-phase components (contains solid oil, liquid oil, and powder) were mixed in the preparation of the oil-in-water emulsion composition. The oil-in-water emulsion composition of Comparative Example 1 also had a slightly inferior feeling of use.

Although the embodiments have been described above, the above embodiments are presented as examples, and the present invention is not limited by the above embodiments. The above embodiments can be implemented in various other forms, and various combinations, omissions, substitutions, changes, and the like can be made without departing from the scope of the invention. These embodiments and variations thereof are included in the scope and gist of the invention, and are also included in the inventions described in the claims and their equivalent scope.

The present application claims priority to Japanese Patent Application No. 2022-111422 filed July 11, 2022, the entire contents of which are incorporated herein by reference.

## Claims

1. An oil-in-water emulsion composition in which oil-based particles that contain solid oil, liquid oil, and powder are dispersed in an aqueous phase, wherein
the solid oil consists of naturally derived wax having a melting point of 50°C or more,
the liquid oil contains polar oil and hydrocarbon oil having a branched structure, and
a mass ratio of the naturally derived wax to the hydrocarbon oil is 0.01 to 10.

2. The oil-in-water emulsion composition according to claim 1, wherein a content of the powder is 0.01% to 30% with respect to a total amount of the naturally derived wax and the hydrocarbon oil.

3. The oil-in-water emulsion composition according to claim 1 or 2, wherein the hydrocarbon oil has a carbon number of 10 to 40.

4. The oil-in-water emulsion composition according to claim 3, wherein the naturally derived wax is candelilla wax or carnauba wax.

5. The oil-in-water emulsion composition according to claim 4, wherein the powder is hydrophobic powder.

6. The oil-in-water emulsion composition according to claim 5, wherein the powder contains an amino acid, an amino acid derivative, or powder coated with the amino acid or the amino acid derivative.

7. The oil-in-water emulsion composition according to claim 6, wherein the amino acid derivative is lauroyl lysine.

8. The oil-in-water emulsion composition according to claim 7, wherein the liquid oil does not contain silicone oil.

9. The oil-in-water emulsion composition according to claim 8, wherein
the oil-in-water emulsion composition contains an ultraviolet scattering agent, and an upper limit of a content of the ultraviolet scattering agent is 1 mass% with respect to the oil-in-water emulsion composition.

10. The oil-in-water emulsion composition according to claim 8, wherein the oil-based particles have an average particle size of 50 µm to 10 mm.
